# FASCICULE DE BREVET EUROPEEN

(11) **EP 2 703 364 B1**
(45) Date de publication et mention de la délivrance du brevet: **09.05.2018**
(21) Numéro de dépôt: 13182311.4
(22) Date de dépôt: 30.08.2013
(51) Int. Cl.: C02F 11/04, C02F 3/28, C02F 3/34, C02F 101/10

(54) **Installation démontable pour la production de biogaz**
Zerlegbare Anlage zur Biogaserzeugung
Knockdown facility for the production of biogas

(30) Priorité: 31.08.2012 FR 1258131
(43) Date de publication de la demande: 05.03.2014
(73) Titulaire: Arcbiogaz, 47260 Castelmoron-Sur-Lot (FR)
(72) Inventeur: FAURE, Jean-Marie, 47260 CASTELMORON-SUR-LOT (FR); FAURE, Benoit, 47260 CASTELMORON-SUR-LOT (FR)
(74) Mandataire: Ipside

(56) Documents cités:
- EP-A1- 0 098 023
- EP-A1- 2 130 618
- EP-A1- 2 441 509
- EP-A2- 1 354 940
- WO-A1-82/00299
- WO-A1-2011/072369
- WO-A2-2006/124781
- FR-A1- 2 924 441
- US-A- 4 100 023
- US-B1- 7 186 339

## Description

### DOMAINE DE L'INVENTION

L'invention se rapporte au domaine général de la génération de Biogaz. Il concerne plus particulièrement le domaine de la production de méthane à partir des déchets agricoles, des déchets végétaux ou tous autres déchets organiques méthanisables.

### CONTEXTE DE L'INVENTION - ART ANTERIEUR

En France, la généralisation des installations de production de biogaz se heurte à un problème de prix de revient. L'investissement que représente une telle installation, telle qu'elle est actuellement réalisée, est difficile à rentabiliser.

La meilleure façon de rentabiliser une installation consiste à l'heure actuelle à produire de l'électricité à partir du biogaz produit, électricité qui est ensuite revendue à prix préférentiel à EDF.

Cependant le prix de rachat par EDF du KWh électrique produit dépend de la puissance électrique de l'installation, du rendement global (bilan chaleur - électricité) et d'une prime liée au taux de produits polluants utilisés pour produire le Méthane.

Par suite, pour être rentable, une installation, dans sa forme actuelle, doit avoir une taille importante. Elle doit produire une quantité de gaz suffisante, typiquement, pour produire de manière continue 1MW d'énergie secondaire, de l'énergie électrique ici. Or la construction d'une installation capable de produire 1MW électriques est d'un coût élevé, de l'ordre de 5 M€, ce coût étant largement dû au coût des cuves installées (cuves en béton) et au génie civil nécessaire pour réaliser ces installations.

Or, à l'heure actuelle en France, une activité d'élevage standard produit généralement, en termes de déchets végétaux transformables, une quantité de déchets permettant seulement de produire environ 75 KW électriques. Ainsi une installation de production de biogaz est, dans sa structure actuelle, soit correctement dimensionnée mais non rentable car de capacité de production insuffisante, soit surdimensionnée et par suite sous-exploitée avec les ressources en déchets propres à l'exploitation et donc également non rentable. En conséquence pour une exploitation agricole de taille standard l'investissement nécessaire pour mettre en place une unité de cogénération de biogaz et d'électricité est non rentable car impossible à amortir (balance investissement - profit d'exploitation jamais en équilibre). C'est pourquoi les installations de production et de transformation de biogaz « à la ferme » sont peu développées en France.

Une telle situation que l'on constate en France est susceptible d'être rencontrée dans d'autres pays.

Or, malgré tout, le méthane représente une source d'énergie relativement avantageuse dans la mesure où les rejets de combustion sont essentiellement constitués de CO₂ et de vapeur d'eau, le biogaz produit à partir de déchets végétaux étant, quant à lui, composé essentiellement de Méthane et de CO₂. Par suite, le biogaz utilisé par un cogénérateur, ou, plus généralement par un moteur thermique destiné à un usage quelconque, ne produit pas de gaz à effet de serre, le CO2 produit rentrant dans le cycle naturel du carbone à travers la photosynthèse. Il en est de même pour la vapeur d'eau.

Par ailleurs, l'utilisation d'une installation de production de biogaz permet la valorisation locale des déchets produits par l'exploitation déchets qui dans tous les cas doivent être éliminés, avec ou sans valorisation.

C'est pourquoi, il existe un besoin réel de disposer d'installations moins coûteuses à fabriquer et à mettre en service.

WO2006/124781 divulgue une installation de production de biogaz, fonctionnant en mode continu, comportant plusieurs cuves à réaction fonctionnant en série.

### PRESENTATION DE L'INVENTION

Un but de l'invention est de proposer une solution permettant d'abaisser le coût d'acquisition, de mise en service et de maintenance d'une installation de production de biogaz (Méthane essentiellement) à partir de déchets organiques, des déchets végétaux en particulier, de telle façon que pour une puissance délivrée maximum donnée on puisse rentabiliser les investissements engagés.

Un autre but de l'invention est de proposer une solution permettant de disposer d'une installation de production de biogaz qui puisse être démontée de manière simple, de façon à être éventuellement réutilisée sur un autre site, sans laisser subsister, après son démontage, d'infrastructures lourdes dont la démolition serait coûteuse.

Un autre but encore consiste à proposer une solution présentant un rendement maximum.

A cet effet l'invention a pour objet une installation selon la revendication 1. D'autres caractéristique complémentaire sont l'objet des revendications dépendante de cette revendication.

### DESCRIPTION DES FIGURES

Les caractéristiques et avantages de l'invention seront mieux appréciés grâce à la description qui suit, description qui s'appuie sur les figures annexées qui présentent:
- la figure1, une vue schématique générale des principaux éléments de l'installation selon l'invention ;
- la figure 2, une vue plane présentant une cuve de production de biogaz selon l'invention ;

- la figure 3, une représentation schématique présentant les différents éléments de l'installation selon l'invention et les différentes canalisations reliant ces éléments ;
- la figure 4, un vue schématique partielle, en coupe, de la pompe de recirculation de l'installation selon l'invention ;
- la figure 5, un ensemble de vues présentant un dispositif d'alimentation en matière organique sèche permettant d'alimenter la pré-cuve de l'installation selon l'invention ;
- la figure 6, une illustration schématique présentant l'association du dispositif d'alimentation de la figure 5 et de la pré-cuve.

### DESCRIPTION DETAILLEE

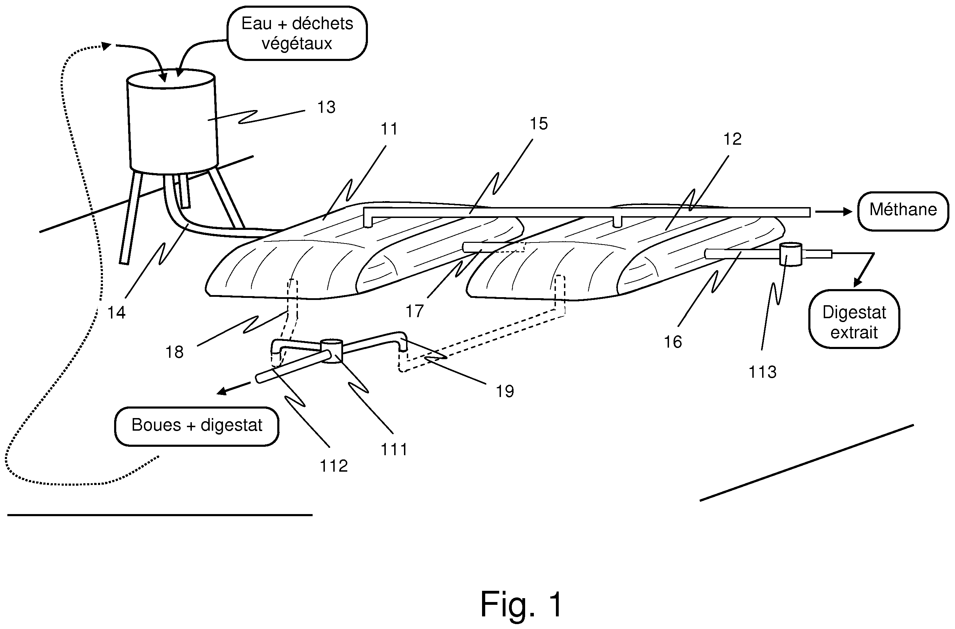

Les figures 1 à 6 illustrent au travers de représentations schématiques les caractéristiques de l'installation selon l'invention. Il est à noter ici que les représentations schématiques constituant ces figures ne constituent pas des représentations à l'échelle.

Les figures présentées illustrent l'invention au travers d'un exemple de mise en oeuvre qui correspond à une exploitation agricole. La présentation détaillée de cet exemple de mise en oeuvre a pour but de mettre en évidence les caractéristiques techniques de l'invention de manière claire. Elle n'est cependant aucunement limitative de la portée ou du champ d'application, du domaine d'emploi, de l'invention. Il est particulier entendu que l'emploi de l'installation selon l'invention ne se limite pas à la méthanisation de déchets végétaux mais peut être généralisé à la méthanisation de tous types de déchets organiques méthanisables dans le contexte d'exploitations diverses, agricoles, alimentaire ou autres.

Comme l'illustre la figure 1 l'installation selon l'invention comporte cinq éléments principaux :
- une première cuve à fermentation 11, ou digesteur, posée au sol ;
- une seconde cuve à fermentation 12, ou post-digesteur également posée au sol ;
- une cuve de brassage 13, ou pré-cuve, placée en hauteur par rapport au digesteur et au post-digesteur ;
- une pompe de recirculation 111 également posée au niveau du sol;
- une pompe d'extraction 113.

Elle comporte par ailleurs un ensemble de canalisations :
- Une canalisation 14 qui relie la pré-cuve 13 au digesteur 11 ;
- une canalisation 17 qui relie le digesteur 11 au post digesteur 12 ;
- deux canalisations 18 et 19 qui relient respectivement le digesteur11 et le post-digesteur 12 à la pompe de recirculation 111 ;
- Une canalisation 112 qui relie la pompe de recirculation 111 à la pré-cuve 13 ;
- une canalisation 16 qui relie le post-digesteur 12 à la pompe 113 d'extraction ;
- un jeu de canalisations 15 qui collecte le biogaz produit par le digesteur 11 et le post-digesteur 12.

De manière connue le digesteur est un bioréacteur où des microorganismes, ou archées, transforment la fraction « digérable » de la matière organique utilisée (souvent autour de 80% du poids sec de matière) en biogaz. Il en est de même pour le post digesteur, seconde cuve, généralement identique à la première, destinée à prolonger (doubler) le temps de séjour du digestat et à augmenter ainsi la quantité de biogaz récupérée par tonne de matière nouvelle entrante (représente environ 20% de la production totale).

Selon l'invention et à la différence des systèmes analogues existants, les deux cuves de fermentation, le digesteur 11 et le poste digesteur 12, sont des éléments en matériau souple, en matière plastique de type PVC par exemple, formant deux enveloppes dont la mise en forme est réalisée par le digestat contenu dans la partie inférieure de la cuve et le gaz produit qui occupe la partie supérieure. Ces deux enveloppes sont destinées à être posées au sol.

Les deux cuves 11 et 12 ont préférentiellement des dimensions identiques et peuvent prendre, en fonction de la forme de l'enveloppe qui les constitue, une forme globalement parallélépipédique, comme l'illustre la figure 1, ou alternativement une forme cubique, ou encore cylindrique.

De préférence les cuves ainsi formées sont relativement peu profondes, par rapport aux cuves traditionnellement utilisées. Leur hauteur est plus faible que les autres dimensions formant la surface de base, une hauteur hors sol de 1,5 m à 2 m pour une surface de 400 m² par exemple, de sorte que chacune des cuves 11 ou 12 a l'aspect d'un container plat, comme l'illustre la figure 1.

Selon une forme de réalisation particulière de l'invention, prise comme exemple, les cuves à réaction 11 et 12 sont des cuves souples sensiblement parallélépipédiques (20m x 20m x 1,5m) posées à même le sol, qui sont capables de stocker 100 à 150 m³ de biogaz.

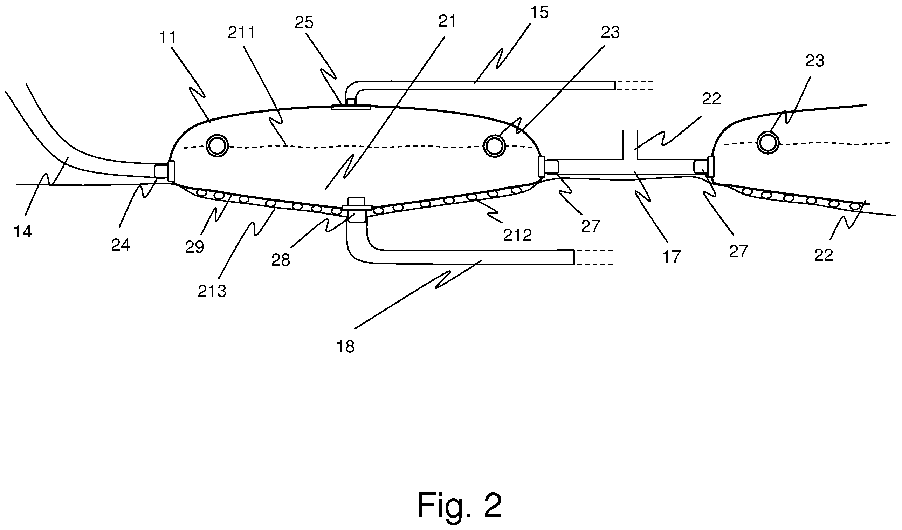

Comme l'illustre la figure 2, chacune des cuves 11 et 12 présente une face inférieure, une base 29, destinée à être posée sur le sol. A cet effet le sol est préparé de façon à former une excavation évasée de forme sensiblement tronc-conique, ou encore de la forme d'une pyramide inversée, présentant un point bas central ; la paroi 213 de l'excavation ainsi formée constituant la surface de pose de la face inférieure de la cuve qui, lorsqu'elle est remplie, en épouse le galbe. Cette face inférieure 29 est munie en son centre d'un embout 28 permettant la fixation d'une canalisation enterrée 18 ou 19, d'un diamètre important, typiquement 300 m, faisant partie du dispositif de recirculation dont est avantageusement équipée l'installation selon l'invention. Cette canalisation chemine dans une tranchée débouchant au centre de l'excavation.

Le dispositif de recirculation comporte également, outre les canalisations 18 et 19, la pompe de recirculation 111 et la canalisation 112 qui relie celle-ci à la pré-cuve 13. Ce dispositif, qui permet d'extraire de chacune des cuves les boues accumulées au fond de la cuve ainsi qu'une partie du digestat en cours de fermentation situé en partie basse de la cuve, comporte en outre une vanne de raccordement à double entrée et à sortie unique.

Chaque entrée est reliée à une des canalisations 18 ou 19, tandis que la sortie est raccordée à l'entrée de la pompe de recirculation 111.

Un moyen de commutation permet de faire circuler dans la pompe soit les matières issues du digesteur 11 soit celles issues du post-digesteur 12, ces matières s'écoulant jusqu'à l'entrée de la pompe 111 par simple gravité.

Selon l'invention les canalisations 18, 19, et 112, de même que les orifices correspondants sur les cuves 11 et 12, ainsi que la pompe de recirculation 111, présentent un diamètre d'écoulement très important, qui permet d'éviter que le dispositif de recirculation, la pompe de recirculation en particulier, ne se bouche du fait de la présence de matières organiques fibreuses.

Ainsi, si on considère les cuves souples prises comme exemple dans le texte qui précède (cuves parallélépipédiques de 20m x 20m x 1,5m) le diamètre des canalisations 18, 19 et 112, ainsi que le diamètre de la pompe est typiquement de l'ordre de 300 mm.

La pompe de recirculation 111 est conçue de façon à présenter un débit important, de l'ordre de 100 à 150 m³ typiquement. Avantageusement du fait de la relativement faible hauteur des cuves constituant le digesteur 11 et le post-digesteur 12, la pression des matières reste relativement faible, de sorte qu'il est possible de mettre en oeuvre une pompe efficace bien que présentant une pression de refoulement relativement faible. En fonctionnement, la pompe de recirculation 111 est conçue pour faire remonter plusieurs mètres cubes de digestat et de boues par heure vers la pré-cuve située en hauteur, 3 m³ par heure typiquement.

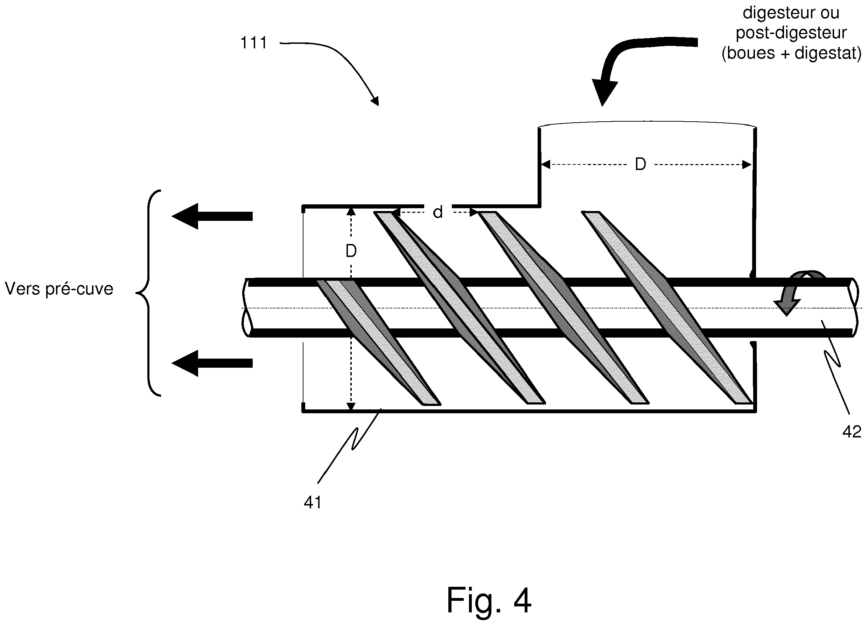

Selon un mode de réalisation préféré, cette pompe se compose, comme l'illustre la figure 4, d'un corps 41 présentant un diamètre D important, à l'intérieur duquel tourne une vis sans fin 42 courte au regard de sa longueur, comportant typiquement 3 spires et demie. La vis sans fin 42 est entraînée en rotation par un moteur non représenté sur la figure 4. Selon l'invention le pas d de la vis sans fin 42 est déterminé de façon à éviter le tassement des matières à l'intérieur de la pompe et à minimiser la puissance nécessaire à l'extraction de ces dernières. Le pas d est également déterminé pour rendre ladite pompe tolérante aux cailloux ou aux corps durs résiduels pouvant se mêler aux boues.

Le dispositif de recirculation décrit dans les paragraphes précédents, dont la mise en place est rendue possible par l'utilisation des cuves souples de relativement faible hauteur qui constituent le digesteur 11 et le post-digesteur 12, constitue un dispositif réellement avantageux, dont ne sont pas pourvues les installations classiques du fait notamment de leur plus grande hauteur, celles équipées de cuves à structure rigide en particulier. Il permet de vidanger les boues déposées au fond de la cuve sans avoir à ouvrir la cuve et par suite à arrêter le fonctionnement du bioréacteur.

Du fait de leur architecture, les méthaniseurs (bioréacteurs) classiques ne comportent pas, quant à eux, de moyens de gestion des inévitables boues et sables présents (en petite quantité) dans les matières entrant dans le digesteur, ou intrants. En conséquence, ces produits indésirables s'accumulent progressivement en fond de cuve, ce qui réduit le volume utile et fini par nuire au rendement du digesteur. Par suite, les cuves doivent être curées périodiquement, de manière relativement fréquente, durant la période d'exploitation des installations, tous les 4 ou 5 ans en moyenne. Outre le fait qu'elle nécessite l'arrêt complet du bioréacteur durant le nettoyage, cette opération, déjà onéreuse en soi, fait aussi perdre plusieurs mois de pleine production du fait notamment du temps nécessaire après remise en service des cuves pour la montée à puissance nominale du bioréacteur.

Il est à noter ici que, du fait de ses caractéristiques, le dispositif de recirculation permet de récupérer non seulement les boues accumulées au fond du digesteur et du post-digesteur mais aussi, et surtout, une partie du digestat contenu dans ces deux cuves, l'ensemble étant acheminé jusqu'à la pré-cuve 13. La recirculation d'une proportion du digestat contenu dans la cuve permet avantageusement, comme cela est détaillé plus loin dans la présente description, de réguler de manière efficace, avec un minimum d'apport d'eau de l'extérieur, le taux de matière sèche, du mélange introduit depuis la pré-cuve 13 dans le digesteur 11 à un instant donné.

Chacune des cuves comporte également, comme l'illustre la figure 2, deux orifices latéraux, placés sensiblement au niveau du sol et pourvu d'une vanne et/ou d'un embout, 24, 26 ou 27, permettant le raccord d'une canalisation 14, 16 ou 17 d'un diamètre important, de l'ordre de 300 mm typiquement, ce diamètre permettant un écoulement fluide de la matière première ou du digestat.

On rappel ici que le digestat est la matière produite au cours de la fermentation des déchets végétaux introduits dans les cuves. Ce digestat est constitué par introduction dans le digesteur 11 d'une matière première constituée de déchets végétaux concassés et mélangé principalement à de l'eau de façon à obtenir la consistance d'une pâte liquide. Sa fermentation anaérobie sous l'action des archées et autres bactéries à l'intérieur des bioréacteurs (digesteur 11 et post-digesteur 12) génère du biogaz (méthane principalement) qui s'accumule dans la partie supérieur de la cuve, tandis que la matière première en cours de fermentation, ou digestat, occupe la partie inférieure.

En ce qui concerne le digesteur 11, un premier orifice est relié à la pré-cuve 13 par l'intermédiaire d'une canalisation 14 dont le diamètre est adapté à cet effet, tandis que le second orifice est relié par l'intermédiaire d'une autre canalisation 17, d'un diamètre également adapté, au post-digesteur 12. Ainsi, les deux cuves 11 et 12 sont reliées l'une à l'autre par un système de vases communicants, la canalisation 17 permettant un passage de digestat du digesteur 11 vers le post-digesteur 12. L'entrée de la canalisation 17 raccordée à l'orifice de sortie du digesteur par l'embout 26 est par ailleurs équipée d'une valve anti reflux interdisant, sur commande, le retour dans le digesteur 11 de digestat contenu dans le post-digesteur 12.

En ce qui concerne le post-digesteur 12, un premier orifice est relié à une canalisation 16, de fort diamètre également, typiquement de l'ordre de 200 mm, permettant d'évacuer une proportion donnée de digestat en dehors de la cuve de façon à maintenir constant le niveau interne de liquide, tandis que le second orifice est relié à la canalisation 17 qui le relie au digesteur 11.

Selon l'invention, la canalisation 17 qui relie le post-digesteur 12 au digesteur 11 peut selon la configuration adoptée être directement posée au sol, comme illustré par la figure 2, ou bien enterrée, de sorte que les orifices permettant de relier le digesteur au post-digesteur sont agencés en conséquence, soit latéralement soit sur la surface inférieure 29. Plus généralement, l'agencement de la canalisation 17 est dans tous les cas défini de telle façon que le passage de matière du digesteur 11 au post-digesteur 12 puisse se faire naturellement suivant le principe des vases communicants.

Selon l'invention également, les parois du digesteur 11 et du post digesteur 12 sont équipés de sas circulaires étanches 23 placées à une hauteur correspondant sensiblement à la hauteur de digestat à l'intérieur de la cuve considérée, c'est à dire au niveau de l'interface digestat/biogaz, matérialisée par le trait pointillé 211 sur la figure 2. Ces sas 23, à la fois étanches aux liquides et aux gaz, permettent l'introduction partielle d'objets à l'intérieur de la cuve.

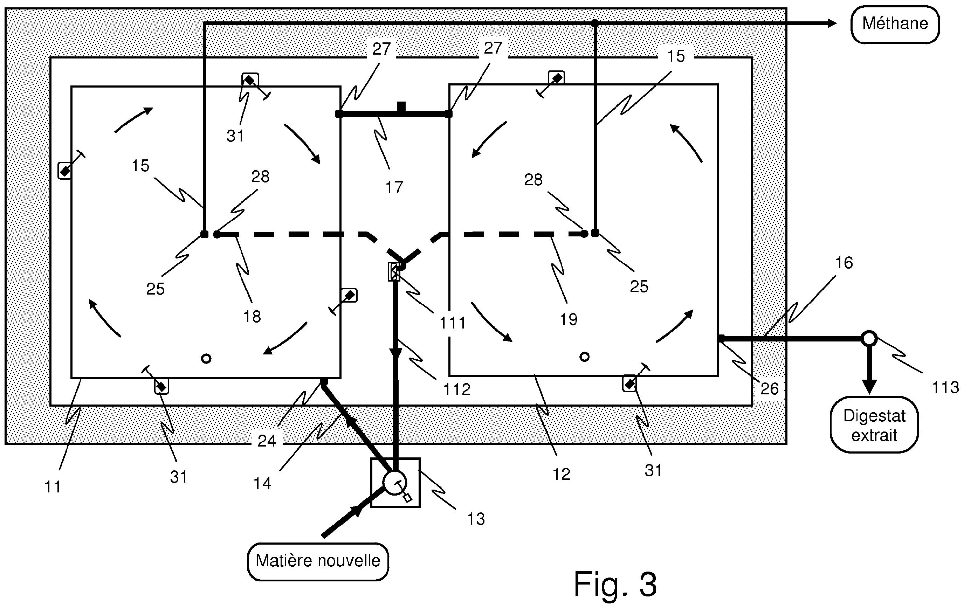

Dans le cas présent les objets sont ici principalement, comme l'illustre la figure 3, des moyens agitateurs 31 réalisant le brassage du digestat. En effet, le contenu des cuves à réactions que constituent le digesteur 11 et le post-digesteur 12, doit être fréquemment mélangé, afin que le digestat soit de structure homogène.

Selon l'invention les moyens de brassage 31 sont constitués de bras agitateurs, mus par l'intermédiaire d'un arbre actionné par un moteur, qui sont plongés dans le digestat à proximité de la surface, l'arbre traversant le sas étanche 23. De la sorte, le moteur est avantageusement placé à l'extérieur de la cuve. Il est ainsi possible d'utiliser un moteur standard conforme à la réglementation ATEX (ATmosphères EXplosive), c'est-à-dire non nécessairement étanche, de puissance appropriée pour réaliser le brassage en surface du digestat.

Les bras agitateurs sont préférentiellement positionnés à l'intérieur de la cuve de façon à générer un courant tournant à l'intérieur du digestat. Le nombre d'agitateurs est fonction de la puissance de chacun des moyens de brassage 31 et de la puissance nécessaire pour mettre et maintenir en mouvement le digestat à l'intérieur de la cuve 11 ou 12. Ces agitateurs sont introduits dans la cuve par les sas étanches 23 qui sont positionnées sur la paroi de façon appropriée, au voisinage de chacun des angles pour des cuves de section carrée ou rectangulaire, ou tous les 90° pour des cuves de section circulaire. On compte ainsi, par exemple, quatre agitateurs pour le digesteur et deux ou quatre agitateurs pour le post-digesteur.

Selon l'invention, les sas étanches 23 sont conçues de façon à maintenir l'étanchéité de la cuve au gaz et au digestat lorsque les arbres de agitateurs les traversent et ce, malgré les vibrations qui sont susceptibles de secouer ledit arbre.

Il est à noter que, le brassage étant une fonction qui ne doit pas tomber en panne sans possibilité de remise en marche pendant toute la durée d'activité de l'installation, de l'ordre 15 ans généralement, l'utilisation de moyens de brassage multiples 31, extractibles de la cuve souple par les sas étanches 23 permet avantageusement, en cas de panne d'un des moyens, de réaliser la maintenance de ce moyen sans avoir à vider la cuve, tandis que les moyens restants maintiennent un certain brassage. A défaut d'un tel agencement on est généralement contraint, pour récupérer et réparer le moyen de brassage en panne, d'ouvrir et de vidanger la cuve touchée, ce qui revient à arrêter complètement la production de biogaz, puis, après réparation de procéder à un remise en production du bioréacteur, lequel ne revient que progressivement à sa puissance nominale.

Chacune des cuves 11 ou 12 comporte également sur sa face supérieure un orifice d'échappement équipé d'un embout 25 permettant au biogaz produit de s'échapper de la cuve. Afin de maintenir une pression constante l'intérieur de la cuve, l'embout 25 de l'orifice d'échappement est avantageusement pourvu d'une soupape permettant l'évacuation du biogaz de l'intérieur de la cuve dès lors que la pression à l'intérieur de cette dernière dépasse une valeur limite donnée ou pression de service. Les orifices d'échappement du biogaz hors des cuves 11 et 12 sont raccordés à un circuit collecteur 15 qui conduit le biogaz vers l'installation de cogénération, chargée de transformer le biogaz produit en énergie, électricité et chaleur principalement.

Il est à noter que, selon l'invention, les cuves souples 11 et 12 formant le digesteur et le post-digesteur ont une épaisseur de peau qui permet de maintenir le biogaz produit à une pression sensiblement plus élevée que dans une cuve en béton classique dotée d'une étanchéité à membrane souple EPDM. De la sorte, la régulation du fonctionnement du bioréacteur est plus facile dans la mesure où les variations de pression de biogaz sont plus faciles à mesurer. On peut ainsi détecter de manière quasi-immédiate les variations de rendement des réactions à l'intérieur de la cuve et prendre de manière tout aussi immédiate les mesures correctrices jugées nécessaires. Par ailleurs, une pression nominale supérieure permet d'absorber des variations plus importantes sans que celle-ci n'affecte la production.

Cependant le biogaz produit est un mélange comportant majoritairement du méthane, mais également des composants indésirables tels que l'eau ou le sulfure d'hydrogène. Ces composants doivent être éliminés en quasi-totalité pour que le biogaz puisse être utilisé en toute sécurité par le cogénérateur.

Classiquement, l'élimination de ces composants est réalisée par l'injection d'air dans le digesteur et le post-digesteur, dans une proportion définie. Cette injection est réalisée directement au niveau l'horizon gazeux de chacune des cuves. Cependant cette façon connue de procéder a pour inconvénient de pénaliser la production de biogaz, réaction anaérobie, la présence d'oxygène étant nuisible aux archées méthanogènes qui se développent à la surface du digestat. Elle a également pour inconvénient de présenter un risque d'explosion dans la partie haute du digesteur où se forme le biogaz, en particulier dans le cas où la proportion d'air injecté s'avère trop importante.

Pour éviter ces inconvénients, l'installation selon l'invention est conçue pour être utilisée avec un dispositif d'épuration externe situé en dehors du digesteur et du post-digesteur et qui n'influe donc pas sur les réactions. En outre, selon une forme de réalisation préférée, le dispositif utilisé, développé par la demanderesse, est un dispositif spécifique, avantageusement simple, constitué d'un long tube sur la paroi interne duquel sont montées des éléments de bois formant des chicanes sur lesquelles se développent des bactéries se nourrissant de sulfure d'hydrogène. Ces chicanes forment une surface de contact importante entre le biogaz et les bactéries, de sorte que le biogaz se décharge de son sulfure d'hydrogène au cours de sa traversée du dispositif. Un tel dispositif externe d'épuration est avantageusement plus simple et moins cher que les dispositifs d'épuration externes disponibles sur le marché.

Il est à noter cependant que le biogaz produit par le bioréacteur étant par principe saturé en eau, il se forme progressivement de l'acide dans le tube, acide qui finit par imprégner le bois des chicanes, ce qui nuit à la bonne santé des bactéries dont les chicanes constituent le milieu. Par suite la filtration devient moins bonne et une proportion de sulfure d'hydrogène reste alors présente dans le biogaz. C'est pourquoi il est nécessaire de surveiller le pH du milieu à l'intérieur du dispositif et en cas de pH trop faible d'intervenir en remplaçant le dispositif usagé par un dispositif neuf, ou alternativement en mettant en place un système de neutralisation de l'acidité du milieu.

Il est à noter que, comme cela a été dit précédemment, que la réaction de fermentation qui produit le biogaz à partir de déchets végétaux est une réaction anaérobie sensiblement neutre d'un point de vue thermique, mais qui connait un rendement optimum pour une température « animale » correspondant par exemple à celle qui règne à l'intérieur de la panse d'une vache. Par suite la production de biogaz est avantageusement accélérée si le contenu des cuves constituées par le digesteur 11 et le post digesteur 12 est porté à la température de réaction optimale, 37°C environ. C'est pourquoi dans une forme de réalisation avantageuse, illustrée par la figure 1, les deux cuves constituant le digesteur 11 et le post-digesteur 12 ne sont pas placées directement à même le sol mais sur un revêtement isolant (non représenté) sur lequel sont disposées les canalisations 212 d'un circuit de chauffage. Par ailleurs, l'isolation des parois et de la partie supérieure de la cuve vis-à-vis des intempéries est renforcée en entourant la cuve, après remplissage d'un revêtement isolant et d'une bâche de protection (non représentée également).

Comme cela a également été dit précédemment, l'installation selon l'invention comporte aussi, outre le digesteur 11 et le post-digesteur 12 une pré-cuve 14, comme illustré par la figure 1.

Cette pré-cuve a pour principale fonction de fournir au digesteur 11 une matière première se présentant en phase liquide, c'est-à-dire sous la forme d'une purée fluide, d'une soupe, et ce, quelque soit le type de déchet végétal utilisé, y compris des déchets constitués de fibres longues.

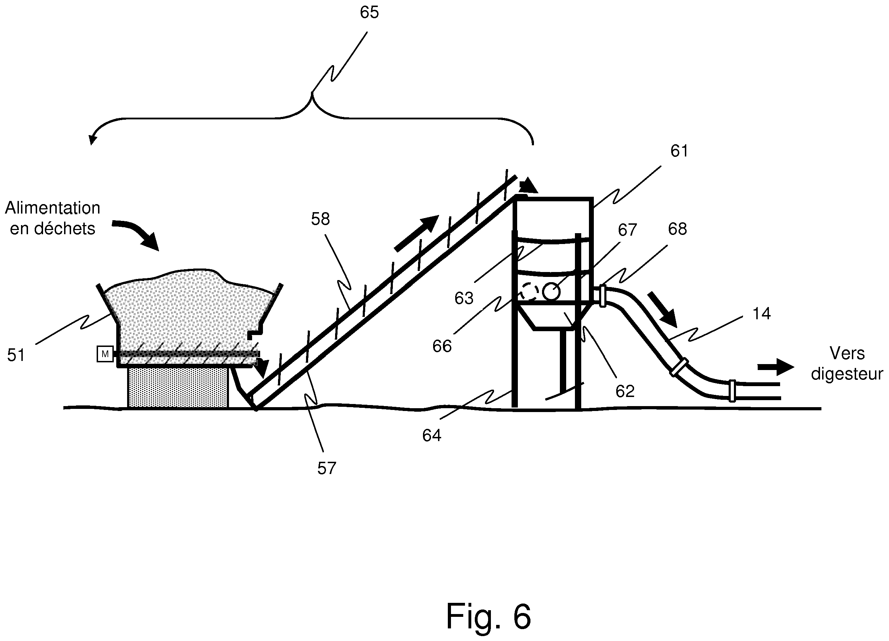

La pré-cuve 13 comporte pour sa part, comme l'illustre la figure 6, une cuve cylindrique 61 d'un volume donné, 4 m³ par exemple, placée en hauteur, ouverte dans sa partie basse sur un embout 62 ayant la forme d'un cône dont la pointe, dirigée vers le bas, est équipée d'une ouverture commandable. Cette cuve est montée sur un support 63 dont les montants 114 reposent sur des pesons qui permettent de connaître l'état de remplissage de la pré-cuve. Elle est, par ailleurs munie d'un mixeur (non représenté sur la figure), positionné en partie haute, qui réalise une homogénéisation du mélange entrant de façon à ce que la matière première ait l'aspect et la consistance voulu.

Ce mélange est constitué de matière nouvelle, des déchets végétaux sec principalement fournis par un dispositif d'alimentation 65, de digestat prélevé dans les cuves 11 et 12 et envoyé à la pré-cuve 13 par la pompe d'extraction 111 au travers d'une canalisation 112 de diamètre approprié, et éventuellement d'un apport de lixiviat fourni par l'exploitation ou d'eau, ces composants étant mélangés dans des proportions variables, ces proportions étant définies de façon à ce que la teneur en matière sèche du mélange formant la matière première introduite dans le digesteur ait une valeur déterminée favorisant l'activité des archées responsables de la production de biogaz.

A cet effet, la pré-cuve 13 comporte donc un orifice d'entrée 66 pour le digestat auquel est raccordée l'extrémité de la canalisation 112 du dispositif de recirculation et une arrivée d'eau 67, l'alimentation en matière nouvelle se faisant généralement par le dessus de la cuve, comme illustré par la figure 6. Elle comporte également une sortie principale 68 destinée à l'alimentation du digesteur. Elle possède en outre aussi une sortie secondaire, située en bas du tronc de cône 62 qui forme la partie basse de la pré-cuve dans laquelle se déposent en grande partie les boues et les cailloux présents dans le mélange en cours de formation. Ce dispositif permet ainsi le stockage en partie basse de la pré-cuve des cailloux et boues denses au cours du malaxage puis leur évacuation.

Il est à noter que, d'un point de vue fonctionnel, sables et boues sont captés pour une large part dans la pré-cuve 13 à leur arrivée avec la matière fraîche, des déchets végétaux principalement. La proportion de ces éléments qui échappe à ce captage est introduite dans le digesteur 11 et dans une plus faible proportion dans le post-digesteur 12, et va se déposer par décantation en fond de cuve, puis passer par l'orifice situé en fond de cuve pour rejoindre la pré-cuve 13 à l'occasion d'un cycle de recirculation, où une partie de cette proportion de boues et de sable boues introduite initialement dans le digesteur 11 sera à son tour piégée. Cette recirculation se reproduit périodiquement, en moyenne plus d'une dizaine de fois pour chaque volume d'intrant, ce qui permettant l'élimination quasi complète, à terme, des boues et sables. Seul une très faible proportion de matière indésirable, les particules fines capables de rester en suspension dans la matière première ou le digestat, est susceptible d'échapper à son élimination et sort finalement du bioréacteur avec le digestat par la canalisation 16 et la pompe d'extraction 113 prévues à cet effet. Le dispositif de recirculation réalise donc de manière efficace, sans nécessiter d'arrêt du fonctionnement de l'installation, l'épuration du digestat contenu dans les cuves.

D'un point de vue dimensionnel, la hauteur du support 63 est définie de façon à ce que la pré-cuve 13 soit positionné au dessus du sol à une hauteur telle que, compte tenu de la hauteur du digesteur, la matière première qu'elle contient puisse se déverser dans le digesteur à travers la canalisation 14 par la simple action de la gravité, le diamètre de la canalisation 14 étant déterminé à cet effet. Cette hauteur est cependant défini en tenant compte de la faible pression de la pompe de recirculation 111, de façon à ce que cette dernière puisse faire remonter le digestat et les boues prélevés dans le digesteur 11 ou le post-digesteur 12 jusqu'à la pré-cuve.

De même, le diamètre de la canalisation 112 et de l'orifice d'entrée 66 sont définis, en fonction de la densité du mélange boue+digestat, de façon à faciliter la remontée de celui-ci dans la pré-cuve 13 par la pompe de recirculation 111, elle-même située au niveau du sol.

L'installation selon l'invention, telle qu'elle est décrite dans le texte qui précède, est conçue pour fonctionner de manière automatique avec une intervention minimum d'un opérateur.

A cet effet dans un mode de mise en oeuvre préféré, elle comporte des moyens de commande et de contrôle dont la fonction consiste principalement à assurer une alimentation régulière de la pré-cuve 13 en matière organique nouvelle, et un mélange d'eau, de matière sèche et de digestat présentant des proportions données permettant de maintenir une pression donnée de biogaz et de conserver un débit constant de biogaz délivré.

Il est à noter que la qualité de régulation du taux de matière sèche tant en entrée de digesteur que dans le digesteur et le post-digesteur est un point important, concernant le fonctionnement d'un bioréacteur. En effet, si ce taux est trop bas, la production de gaz sera insuffisante et le réacteur ne fournira pas le volume de gaz attendu. A contrario, un taux trop élevé peut engendrer des problèmes divers pouvant aller de la difficulté à maintenir un mélange homogène jusqu'à une baisse de rendement, voire, à l'extrême, au blocage de la bioréaction.

Sachant que cette régulation est très importante, des moyens d'action divers sont généralement mis en oeuvre dans les installations de méthanisation.

L'installation selon l'invention dispose quant à elle, par l'introduction de digestat dans la pré-cuve 13, d'un moyen qui augmente les possibilités de régulation du taux de matière sèche. De manière avantageuse, la recirculation partielle de digestat peut être réalisée à partir de digestat issu du digesteur 11 ou bien du post-digesteur 12. On peut ainsi avantageusement choisir de renvoyer dans la pré-cuve un digestat plus ou moins chargé en matière sèche selon l'objectif de régulation poursuivi.

Pour remplir leurs fonctions, les moyens de commande et de contrôle comportent divers actionneurs aptes à mettre en action, aux moments appropriés, la pompe de recirculation 111 ainsi que le malaxeur contenu dans la pré-cuve, les différents mixers 31 logés dans le digesteur 11 et le post-digesteur 12 ainsi que la vanne permettant de diriger soit du digestat issu du digesteur 11 soit du digestat issu du post digesteur 12 à l'entrée de la pompe de recirculation 111 ou la vanne permettant d'empêcher le reflux de digestat du post-digesteur 12 vers le digesteur 11, en particulier lorsque le post digesteur a été vidé d'une partie de son contenu lors d'un cycle de recirculation.

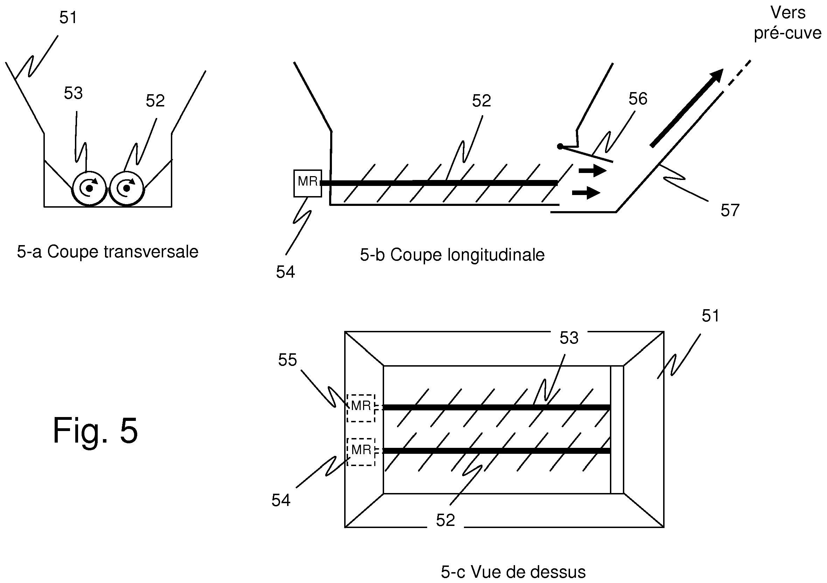

Ces moyens de commande et de contrôle sont associés à un dispositif de chargement automatique de la pré-cuve en matière sèche. Un exemple de dispositif de chargement automatique 65 est présenté sur les figures 5 et 6.

Ce dispositif se compose par exemple d'une trémie 51 fixe ou mobile (sur remorque), dans lequel les différents composants de la « ration journalière » du méthaniseur (bioréacteur) sont versés au moyen d'un tracteur classique muni d'un chargeur, ou encore d'un chargeur télescopique. A cet effet la trémie 51 présente un volume suffisant pour contenir cette ration journalière, un volume d'environ 20 m3 typiquement. Outre sa fonction de stockage, cette trémie a également pour fonction de réaliser une première homogénéisation de la matière entrante (suppression des « paquets »). Elle comporte en partie basse 2 vis sans fin 52 et 53, entraînées chacune par un motoréducteur 54 ou 55, qui s'étendent sur toute sa longueur. Selon la forme de mise en oeuvre considérée la trémie 51 peut fonctionner de manière autonome ou, alternativement être pilotée par les moyens de commande et de contrôle.

Elle est munie à l'arrière d'une porte 56 s'ouvrant sous la poussée de la matière, ladite porte ouvrant sur un dispositif 57 de collecte et de transport de la matière sèche déversée vers la pré-cuve 13. Ce dispositif 57 peut consister en une auge dans laquelle est logée une vis sans fin 58 qui monte la matière à une hauteur suffisante pour que l'entrée de la matière dans la pré-cuve 13 se fasse par gravité, comme illustré par la figure 6. Alternativement il peut consister en une goulotte au fond de laquelle est placé un tapis roulant jouant un rôle analogue à la vis sans fin 58. Le dispositif 65 constitué par la trémie 51 et le dispositif de collecte et de transport 57 permet d'alimenter régulièrement la pré-cuve 13, typiquement une fois par heure, le digesteur 11 tout en en limitant le nombre d'interventions opérées par l'exploitant sur cette partie de l'installation à une intervention de rechargement de la trémie 51 par jour. Il peut en outre être facilement commandé par les moyens de commande et de contrôle. Ainsi, à l'exception de cette action quotidienne de rechargement de la trémie 51, réalisée par l'exploitant, l'ensemble de l'installation peut être configuré de façon à fonctionner de manière automatique sous le contrôle des moyens de commande et de contrôle.

Il est à noter ici, que, de manière générale, les moyens de commande et de contrôle sont constitués, de manière classique pour le pilotage d'un processus, d'un ensemble d'actionneurs chargés de mettre en oeuvre les différents moteurs et vannes de l'installation, et de capteurs chargés de déterminer l'état de cette installation, ces différents éléments étant au service d'un automate. Cet ensemble, de structure classique, dont la fonction est de remplacer une intervention humaine, n'est, pour un souci de simplicité, pas représenté sur les figures accompagnant la description. Il n'en fait cependant pas moins partie de l'installation, dans le cas où cette dernière est destinée à une exploitation automatique.

## Revendications

1. - Installation pour production anaérobie de méthane à partir d'un mélange constitué de déchets organiques, d'eau pure ou de lixiviats comportant :
une cuve de réaction primaire (11) ou digesteur et une cuve de réaction secondaire (12) ou post-digesteur, configurées pour produire du méthane par fermentation anaérobie des déchets organiques, ainsi qu'un résidu organique ou digestat,
un jeu de canalisations (17) reliant les deux cuves de réaction entre elles,
une canalisation (16) reliant le post-digesteur à une pompe d'extraction (113);
une pré-cuve (13), placée en hauteur par rapport au digesteur et au post-digesteur, dans laquelle est préparé le mélange alimentant les cuves de réaction (11, 12), la pré-cuve étant reliée au digesteur par l'intermédiaire d'une canalisation (14),
une pompe de recirculation (111),
un jeu de canalisations (18, 19, 112) reliant les deux cuves (11, 12) à la pompe de recirculation et la pompe de recirculation (111) à la pré-cuve (13);
**caractérisé en ce que** le digesteur (11) et le post digesteur (12) étant constitués chacun par une enveloppe souple étanche, en matière plastique, et configurés pour être posés au sol, les faces inférieures (29) des enveloppes-formant lesdits digesteurs sont pourvues chacune en leur centre d'une ouverture (28) reliée par l'intermédiaire d'une canalisations (18, 19) à la pompe de recirculation (111); les diamètres de l'ouverture (28), des canalisations (18, 19) et de la pompe de circulation (111) étant déterminés de façon à faciliter l'écoulement hors du digesteur (11) et du post-digesteur (12) vers la pompe de recirculation du sable et des boues accumulés au fond des cuves (11, 12) et d'une partie du digestat produit pendant leur fonctionnement, et leur transfert vers la pré-cuve (13) par l'intermédiaire d'une canalisation (112), les canalisations (18, 19) et la pompe de recirculation (111) étant agencées vis-à-vis des cuves (11, 12) de façon à ce que les matières extraites de ces dernières s'écoulent vers la pompe de recirculation(111), et soient évacuées vers la pré-cuve (13) au travers de la canalisation (112) reliant la pompe de recirculation à la pré-cuve; la canalisation (16) et la pompe d'extraction (113) reliées au post-digesteur (12) étant configurées pour permettre l'extraction du digestat contenu dans ce dernier.

2. - Installation selon la revendication 1, **caractérisée en ce que** le digesteur (11) et le post digesteur (12) sont reliés entre eux par une canalisation (17) configurée et agencée de façon à ce que le passage de digestat du digesteur (11) vers le post-digesteur (12) se produise selon le principe des vases communicants lorsque le niveau de matière du post-digesteur est plus bas que celui du digesteur.

3. - Installation selon l'une des revendications 1 ou 2, **caractérisée en ce que** la pré-cuve (13) étant configurée pour produire un mélange composé de matière organique nouvelle, de digestat, d'eau ou de lixiviat, elle comporte un malaxeur qui mixe les produits entrants qui sont introduits de façon à former un mélange ayant la forme d'une pâte fluide.

4. - Installation selon la revendication 3, **caractérisée en ce que** la pré-cuve (13) étant placée en hauteur par rapport au niveau du sol, le diamètre de la canalisation (14) reliant la pré-cuve (13) au digesteur (11) est défini de façon à ce que le mélange préparé dans la pré-cuve (13) puisse être introduit dans le digesteur (11) par simple gravité.

5. - Installation selon l'une des revendications 3 ou 4, **caractérisée en ce que** la pré-cuve (13) étant de forme cylindrique, elle comporte un fond (62) de forme sensiblement tronc-conique dans lequel les boues mélangées au digestat réintroduit par la pompe de recirculation (111) se déposent par décantation au cours du malaxage de son contenu.

6. - Installation selon la revendication 5, **caractérisée en ce que** le fond (62) de la pré-cuve (13) comporte un orifice obturé par une vanne par lequel les boues décantées sont extraites de la pré-cuve.

7. - Installation selon l'une quelconque des revendications 1 à 6, **caractérisée en ce que** le digesteur (11) et le post digesteur (12) étant des cuves de faible hauteur, la pompe de recirculation (111) est une pompe à grand débit et à faible pression configurée pour être apte à remonter le mélange de boues et de digestat prélevé dans le digesteur (11) ou le post-digesteur (12) vers la pré-cuve (13).

8. - Installation selon la revendication 7, **caractérisée en ce que** la pompe de recirculation (111) comporte une vis sans fin courte (42), dont le diamètre D et le pas d sont définis de façon à permettre un écoulement libre, sans tassement, du mélange de boues et de digestat prélevé dans le digesteur (11) ou le post-digesteur (12).

9. - Installation selon l'une quelconque des revendications précédentes, **caractérisée en ce qu'**elle comporte en outre des moyens (51-58) d'approvisionnement automatique de la pré-cuve (13) en matières organiques fraîches.

10. - Installation selon l'une quelconque de revendications précédentes, **caractérisée en ce qu'**elle comporte des moyens de commande et de contrôle configurés pour maintenir constant le volume de biogaz produit, les moyens de contrôle agissant sur la teneur en matière sèche du produit de mélange introduit dans le digesteur, la teneur en matière sèche étant régulée en agissant d'une part sur le dispositif de recirculation (111) de façon à introduire en quantité plus ou moins importante soit de digestat extrait du digesteur (11), soit de digestat extrait du post-digesteur (12) et d'autre part sur l'alimentation de la pré-cuve (13) en matière organique nouvelle.

11. - Installation selon l'une quelconque de revendications précédentes, **caractérisée en ce qu'**elle comporte en outre des moyens pour purifier le biogaz produit par le bioréacteur, ces moyens étant constitués de chicanes en bois disposées à l'intérieur de la canalisation (15) qui collecte le biogaz produit par le digesteur (11) et le post digesteur (12).

## Patentansprüche

1. - Vorrichtung für die anaerobe Gewinnung von Methan aus einer Mischung von Bioabfall, reinem Wasser oder Sickerwässern, bestehend aus:
ein Tank zur primären Reaktion (11) oder Fermenter und ein Tank für die zweite Reaktion (12) oder Nachfermenter, konfiguriert zur Gewinnung von Methan durch anaerobe Fermentierung von Bioabfall, und ein biologischer Reststoff oder Gärreste
ein Satz Rohre (17), die die beiden Reaktionstanks miteinander verbinden,
ein Rohr (16), das den Nachfermenter mit einer Extraktionspumpe verbindet (113);
eine Vorgrube (13), in einer Höhe in Beziehung zum Fermenter und Nachfermenter platziert, in der die Mischung vorbereitet wird, die die Reaktionstanks auffüllt (11, 12), die Vorgrube ist mit dem Fermenter durch ein Rohr verbunden (14),
eine Umwälzpumpe (111)
ein Satz Rohre (18, 19 112) der die zwei Tanks (11, 12) mit der Umwälzpumpe verbindet und die Umwälzpumpe (111) mit der Vorgrube (13);
**gekennzeichnet durch** den Fakt, dass der Fermenter (11) und der Nachfermenter (12), jeweils aus einer flexiblen wasserdichten Hülle aus Kunststoff hergestellt sind und so eingerichtet wurden, dass sie auf dem Boden stehen, die Unterseiten (29) der Hüllen, die besagte Fermenter bilden, sind in ihrer jeweiligen Mitte mit einer Öffnung ausgestattet (28) und über ein Rohr (18, 19) mit der Umwälzpumpe verbunden (111); die Durchmesser der Öffnung (28) der Rohre (18, 19) und der Umwälzpumpe 111) sind so festgelegt, dass der Fluss außerhalb des Fermenters (11) ermöglicht wird und beim Nachfermenter (12) in Richtung Umwälzpumpe zum Sand und Schlamm hin, die sich am Boden des Tanks ansammeln (11, 12) und ein Teil des Gärrests, der während ihres Betriebs produziert und zur Vorgrube (13) mittels eines Rohres (112) weitergeleitet wird, die Rohre (18, 19) und die Umwälzpumpe (111) sind gegenüber den Tanks (11, 12) derart angeordnet, dass die Materialien, die aus diesem Fluss in Richtung Umwälzpumpe (111) herausgezogen werden und zur Vorgrube (13) durch das Rohr (112), das die Umwälzpumpe mit der Vorgrube verbindet, entleert werden; das Rohr (16) und die Extraktionspumpe (113), die mit dem Nachfermenter (12) verbunden sind, sind so konfiguriert, dass die Extraktion des Gärrests, der in Letzterem enthalten ist, ermöglicht wird.

2. - Vorrichtung nach Patentanspruch 1, **gekennzeichnet dadurch, dass** der Fermenter (11) und der Nachfermenter (12) über ein Rohr (17) verbunden sind, konfiguriert und so angeordnet, dass der Durchfluss des Gärrests vom Fermenter (11) zum Nachfermenter (12) nach dem Prinzip kommunizierender Röhren stattfindet, wenn der Pegel des Materials im Nachfermenter niedriger ist als im Fermenter.

3. - Vorrichtung entsprechend einem der Patentansprüche 1 oder 2, **gekennzeichnet dadurch, dass** die Vorgrube (13) so konfiguriert ist, dass eine Mischung von neuem Biomaterial, Gärrest, Wasser oder Sickerwasser erzeugt wird, bestehend aus einem Mischer, der hereinkommende Produkte mischt, die eingeleitet wurden, um eine Mischung in Form einer flüssigen Paste zu bilden.

4. - Vorrichtung entsprechend Patentanspruch 3, **gekennzeichnet dadurch, dass** die Vorgrube (13) in einer Höhe relative zur Bodenhöhe platziert wird, der Durchmesser des Rohres (14), das die Vorgrube (13) mit dem Fermenter (11) verbindet, ist festgelegt, damit die in der Vorgrube (13) vorbereitete Mischung einfach durch Schwerkraft in den Fermenter (11) eingeleitet werden kann.

5. - Vorrichtung entsprechend einem der Patentansprüche 3 oder 4, **gekennzeichnet dadurch, dass** die Vorgrube (13) eine zylindrische Form hat, am Boden (62) aus einer erheblich gekürzten Kegelform besteht, in der der Schlamm mit dem Gärrest gemischt wird, welcher durch die Umwälzpumpe (111) wieder eingeführt wird, und sich durch Abgießen während der Mischung seines Inhalts absetzt.

6. - Vorrichtung entsprechend Patentanspruch 5, **gekennzeichnet dadurch, dass** der Boden (62) der Vorgrube (13) aus einer Öffnung besteht, die durch ein Ventil geschlossen wird, durch welches der Schlamm aus der Vorgrube extrahiert wird.

7. - Vorrichtung entsprechend einem der Patentansprüche 1 bis 6, **gekennzeichnet dadurch, dass** der Fermenter (11) und der Nachfermenter (12) Tanks von geringer Höhe sind, die Umwälzpumpe (111) eine Niederdruckpumpe mit hohem Durchfluss ist und so konfiguriert, dass sie die Mischung von Schlamm und Gärrest anheben kann, die aus dem Fermenter (11) oder dem Nachfermenter (12) in Richtung Vorgrube (13) gezogen wird.

8. - Vorrichtung entsprechend Patentanspruch 7, **gekennzeichnet dadurch, dass** die Umwälzpumpe (111) eine kurze Schnecke (42) enthält, der Durchmesser D und der Abstand d davon sind so festgelegt, dass ein freier Fluss der Mischung aus Schlamm und Gärrest, die von dem Fermenter (11) oder dem Nachfermenter (12) genommen wird, ohne Sackung möglich ist.

9. - Vorrichtung entsprechend einem der vorherigen Patentansprüche ist **gekennzeichnet dadurch, dass** sie Mittel (51-58) für die automatische Belieferung der Vorgrube (13) mit frischen Biomaterialien beinhaltet.

10. - Vorrichtung entsprechend einem der vorherigen Patentansprüche, ist **gekennzeichnet dadurch, dass** sie Mittel zur Führung enthält, entsprechend konfiguriert, dass das Volumen des gewonnenen Biogas konstant bleibt, die Mittel zur Steuerung wirken auf den Trockensubstanzgehalt des Produkts der Mischung, die in den Fermenter eingeführt wird, der Trockensubstanzgehalt wird dadurch reguliert, indem er einesteils auf die Umwälzpumpe (111) einwirkt, um größere oder kleinere Mengen des Gärrests, der aus dem Fermenter (11) extrahiert wird oder des Gärrests aus dem Nachfermenter (12), einzuführen und zweitens durch Einspeisen der Vorgrube (13) mit neuem Biomaterial.

11. - Vorrichtung entsprechend einem der vorherigen Patentansprüche, ist **gekennzeichnet dadurch, dass** sie weiter Mittel zur Reinigung des durch den Bioreaktor gewonnenen Biogases enthält, diese Mittel bestehen aus hölzernen Lenkblechen, die im Rohr (15) angebracht sind, welches das vom Fermenter (11) und Nachfermenter (12) gewonnene Biogas sammelt.

## Claims

1. - Apparatus for anaerobic production of methane from a mixture of organic waste, pure water or of leachates comprising:
a tank for primary reaction (11) or digester and a tank for secondary reaction (12) or post-digester, configured for production of methane by anaerobic fermentation of organic waste, and an organic residue or digestate
a set of pipes (17) connecting the two reaction tanks together,
a pipe (16) connecting the post-digester to an extraction pump (113);
a pre-tank (13), placed at a height in relation to the digester and the post-digester, in which the mixture is prepared that feeds the reaction tanks (11, 12), the pre-tank being connected to the digester through a pipe (14),
a recirculation pump (111)
a set of pipes (18, 19 112) connecting the two tanks (11, 12) to the recirculation pump and the recirculation pump (111) to the Pre-tank (13);
**characterized by** the fact that the digester (11) and the post digester (12), each being constituted by a flexible waterproof envelope, in plastic material, and configured to be placed on the ground, the lower sides (29) of the envelopes forming said digesters are provided each in their center with an opening (28) connected through a pipe (18 , 19) to the recirculation pump (111); the diameters of the opening (28) of the pipes (18, 19) and of the circulation pump 111) being determined so that facilitate the flow outside the digester (11) and the post-digester (12) towards the recirculation pump of the sand and sludge accumulated at the bottom of the tanks (11, 12) and a part of the digestate produced during their functioning and their transfer towards the pre-tank (13) by means of a pipe (112), the pipes (18, 19) and the recirculation pump (111) being arranged vis-a-vis the tanks (11, 12) in such a way that the materials extracted from these flow towards the recirculation pump (111), and are evacuated towards the Pre-tank (13) through the pipe (112) connecting the recirculation pump to the pre-tank; the pipe (16) and the extraction pump (113) connected to the post-digester (12) being configured to allow extraction of the digestate contained in the latter.

2. - Apparatus according to claim 1, **characterized in that** the digester (11) and the post digester (12) are interconnected by a pipe (17) configured and arranged so that the passage of the digestate from the digester (11) to the post-digester (12) occurs according to the principle of communicating vessels when the level of material in the post-digester is lower than that of the digester.

3. - Apparatus according to one of claims 1 or 2, **characterized in that** the pre-tank (13) being configured to produce a mixture of new organic material, digestate, water or leachate, comprises a mixer which mixes incoming products that are introduced to form a mixture in the form of a fluid paste.

4. - Apparatus according to claim 3, **characterized in that** the pre-tank (13) being placed in height relative to the ground level, the diameter of the pipe (14) connecting the pre-tank (13) to the digester (11) is defined so that the mixture prepared in the pre-tank (13) can be introduced into the digester (11) by simple gravity.

5. - Apparatus according to one of claims 3 or 4, **characterized in that** the pre-tank (13), being of cylindrical shape, comprises a bottom (62) of substantially truncated conical shape in which the sludge mixed with the digestate reintroduced by the recirculation pump (111) settles by decantation during the mixing of its contents.

6. - Apparatus according to claim 5, **characterized in that** the bottom (62) of the pre-tank (13) comprises an orifice closed by a valve through which the sludge is extracted from the pre-tank.

7. - Apparatus according to any one of claims 1 to 6, **characterized in that** the digester (11) and the post digester (12) being tanks of low height, the recirculation pump (111) is a high flow and low pressure pump configured to be able to raise the mixture of sludge and digestate withdrawn from the digester (11) or the post-digester (12) towards the pre-tank (13).

8. - Apparatus according to claim 7, **characterized in that** the recirculation pump (111) comprises a short endless screw (42), the diameter D and the pitch d of which are defined to allow a free flow, without settlement, of the mixture of sludge and digestate taken from the digester (11) or the post-digester (12).

9. - Apparatus according to any one of the preceding claims, **characterized in that** it further comprises means (51-58) for automatic supply of the pre-tank (13) with fresh organic materials.

10. - Apparatus according to any one of the preceding claims, **characterized in that** it comprises means of command and control configured to keep the volume of biogas produced constant, the control means acting on the dry matter content of the product of mixture introduced into the digester, the dry matter content being regulated by acting on the one hand on the recirculation device (111) so as to introduce greater or lesser amounts of digestate extracted from the digester (11) or digestate extracted from the post-digester (12) and secondly by feeding the pre-tank (13) with new organic material.

11. - Apparatus according to any one of the preceding claims, **characterized in that** it further comprises means for purifying the biogas produced by the bioreactor, these means being constituted by wooden baffles arranged inside the pipe (15) that collect the biogas produced by the digester (11) and the post digester (12).
